# EUROPEAN PATENT SPECIFICATION

(11) **EP 2 982 242 B1**
(45) Date of publication and mention of the grant of the patent: **24.04.2019**
(21) Application number: 14198488.0
(22) Date of filing: 17.12.2014
(51) Int. Cl.: A01K 67/033

(54) **Mite composition, the rearing thereof and methods for application of the composition in biological control systems.**
Milbenzusammensetzung, Aufzucht davon und Verfahren zur Anwendung der Zusammensetzung in biologischen Kontrollsystemen
Composition d'acariens, élevage correspondant et procédé pour l'application de la composition dans des systèmes de contrôle biologique

(30) Priority: 06.08.2014 EP 14180076
(43) Date of publication of application: 10.02.2016
(73) Proprietor: Agrobio S.L., 04745 El Viso (La Mojonera) - Almeria (ES)
(72) Inventor: Vila Rifá, Enrique, 04745 La Mojonera (Almeria) (ES); Griffiths, Donald Alister, West Drayton Middlesex UB7 7AH (GB)
(74) Representative: Hannke, Christian

(56) References cited:
- WO-A1-2006/071107
- WO-A1-2007/075081
- RASMY A H ET AL: "A NEW DIET FOR REPRODUCTION OF TWO PREDACEOUS MITES AMBLYSEIUS-GOSSIPI AND AGISTEMUS-EXSERTUS ACARI PHYTOSEIIDAE STIGMAEIDAE", ENTOMOPHAGA, LIBRAIRIE LE FRANCOIS, PARIS, FR, vol. 32, no. 3, 1 January 1987 (1987-01-01), pages 277-280, XP008118963, ISSN: 0013-8959
- ELLEN A. M. BEERLING ET AL: "Microsporidiosis in mass-rearings of the predatory mites AMBLISEIUS CUCUMERIS and A. Barkeri (ACARINA: PHYTOSEIIDAE)", PROCEEDINGS IN EXPERIMENTAL AND APPLIED ENTOMOLOGY, vol. 2, 1 February 1991 (1991-02-01), pages 157-162, XP055220301,

## Description

### Field of invention

This invention relates to a mite composition comprising a rearing population of a *phytoseiid* predatory mite species and a population of at least one prey species from the order Astigmata, namely *Tyrolichus casei* Ouds.

Further, the invention relates to a method for rearing a *phytoseiid* predatory mite.

Another further aspect of the invention relates to the use of the mite composition for controlling a crop pest.

The following prior art compositions are known to mention the species *Tyrolichus casei,* Ouds. 1912:
Two European patents - WO 2006/071107 AND WO 2007/075081 do so briefly and ambiguously. Each of their A1 pamphlets contain identical paragraphs, as follow -
"The prior art describes rearing of *Amblyseius cucumeris* and *Amblyseius barkeri* with the aid of a factitious host mite species from the genus *Tyrophagus,* in particular *Tyrophagus putrescentiae, Tyrophagus tropicus, Tyrophagus casei* (Sampson, C. 1998; Jackobson, R.J., 1995; Benninson, J. A., and R. Jackobson, 1991; Karg et al 1987; and GB293890)".

There is no distinction given as to which reference refers to what species, *putrescentiae,* or *casei* or both.

Perusal of the five references show that four of them refer only to the use of *T. putrescentiae* under field conditions. The fifth mentions a species *Tyrophagus casei,* but only in the context of being a possible food for oligophagus predatory mites. The British patent GB 2393809, only discloses that *A. cucumeris* can be reared on *T. putrescentiae.*

The publication Castagnoli et al., 2006 [Redia 89, pp.55-58, *Scanning of astigmatid mite for mass rearing Neoseiulus californicus* (McGregor) (Acari: Phytoseiidae)], describes studies relating to a single *phytoseiid* species, namely *N. californicus,* which was shown to be unable to oviposit (lay eggs) and so a first generation failed to be produced, when offered *T. casei* as the factitious host.

Rasmy et al., 1987 [Anzeiger für Schädlingskunde, Pflanzenschutz, Umweltschutz, 60 (5), pp. 92-94, *Reproductive response and development of three soil predaceous mites, utilizing the acarid mite* Tyrophagus casei *(*=Tyrolichus casei*)* as *an alternate 'diet'*] involved an attempt to rear three mite species, which are not members of the *Phytoseiidae,* but belong to other families in the order *Mesostigmata.* Trials involved small rearing units kept in an incubator, with slices of cheese as the food for *T. casei.* One of the species produced some viable eggs, but the other two failed to develop.

Rasmy et al, further discloses in 1987 [Entomophaga, 32 (3), pp.277-280, *A new diet for reproduction of two predaceous mites,* Amblyseius gossipi and Agistemus exertus, *(Acari; Phytoseiidae, Stigmaeidae*)] experimental data about a combination of predatory mites and the acarid mite *T. casei* as food source. The experimental systems were as in the above publication. It involved two species which predate plant mites, but they are quite unrelated. The *Amblyseius* species belongs to the super family *Phytoseioidea,* and the *Agistemus* from the super family *Raphignathoidea.* The *phytoseiid* species *A. gossipi,* completely failed to develop on *T. casei.* This species was only producing eggs when additional pollen was added to the diet. This strongly suggests that predation did not take place or that the prey was not suitable.

Thus the prior art does not show any mass rearing systems of any *phytoseiid* species which uses *Tyrolichus casei* as the factitious host.

Agricultural crops, especially protected crops, are grown intensively over a relatively short growing season. Pests which invade them almost always result from outside invasions and, because the crop provides a very favorable food supply, they can reach high densities very quickly. Thus, any introduction of a predator aimed at controlling them must be inundative. That is large numbers of predators must be introduced in a regular program of introductions sufficient to keep the pest population below the level at which it can cause economic loss.

Therefore, any commercial rearing of a predator, used inundatively, must be able to produce high numbers of the predator throughout the growing season; nowadays under modern glasshouse regimes this means almost year round. To rear a predator requires that it is provided with sufficient suitable prey for rapid population growth. Preferably any mite composition comprising a predator species and a factitious prey which leads to a viable commercial production.

The most successful commercial productions to date are those in which the prey is a species belonging to the mite order of the *Astigmata.* These are small, soft bodied individuals known collectively as 'stored food' or 'stored product' mites, and as such live on a wide range of foodstuffs, especially those of a farinaceous nature, and are commonly found in stores and domestic dwellings (Hughes, 1976). Using the correct food materials and the right physical conditions these *astigmatid* mites are reared indoors in containers to provide high population concentrations at reasonable cost. Currently, all *phytoseiid* predators being produced commercially are reared on one of a few number of these *astigmatid* species.

A problem to be solved by the present invention is providing a mite composition comprising a *phytoseiid* predator species as a rearing population and a factitious host species as food source, which will provide advantages such as lower costs, faster growth rates and other benefits with respect to the compositions known from the prior art.

Further problems to be solved are finding a viable and reliable method for commercially rearing a *phytoseiid* predatory mite and then using it to control a crop pest.

A solution to this problem has been found in a mite composition comprising a rearing population of a *phytoseiid* predatory mite species and at least one host species wherein the host species is a species from the genus *Tyrolichus.* Such a composition is a major aspect of the present invention.

A further major aspect of the invention is a method for rearing a *phytoseiid* predatory mite according to claim 10.

Another further aspect of the invention is the use of the mite composition for controlling a crop pest according to claim 9.

In an advantageous embodiment of the invention the mite composition comprises: a rearing population of a *phytoseiid* mite species, together with the *astigmatid* species *Tyrolichus casei,* Oudemans, 1910. Thus, in this preferred embodiment the host species from the *Astigmata* is *Tyrolichus casei.*

In a preferred embodiment the mite composition comprises a carrier. In a further preferred embodiment the carrier is a neutral substrate which supports the predators in a three dimensional space. Preferably the carrier will be a three dimensional substrate.

In an even more preferred embodiment the carrier is a three dimensional substrate with surfaces arranged three dimensionally in such a manner that these surfaces form cavities and protrusions which will enable the predator and its prey to move about freely within the substrate.

*Tyrolichus* is a monotypic genus with *casei* as the sole species. It has a relatively wide but rarely recorded geographic distribution. A definitive natural habitat is not known, with only one natural recording from beneath the bark of the stumps of some felled trees in Russia, (Zachvatkin, 1941). Other recordings are mostly from stored foods or insect collections, but it does not have a history of being a serious pest of foodstuffs, except for occasional infestations in cheese, hence the latin species name.

Its body conformation is that of a typical member of the order *Astigmata.* Its general shape and chaetotactic formulation (positioning and number of pairs of setae) resemble that of a *Tyrophagus* species. Because of this some early taxonomists placed it in this genus. For example Hughes (1961) placed it in *Tyrophagus,* but in her 1976 edition of 'The Mites of Stored Foods and Houses' she reverted to the decision of the first authority, namely Oudemans, 1924. Even so, Rasmy (1987) still mistakenly used the name *'Tyrophagus'.*

There are distinct morphological differences which separate these two genera, but at low magnifications the similarity is such that even with the use of a compound microscope misidentifications can occur. In such cases, typically, this taxon will be misidentified as *Tyrophagus putrescentiae.*

The species *T. casei* is larger, more stoutly built and moves much slower than most *astigmatid* members, especially when compared with *Tyrophagus putresecentiae,* or *Carpoglyphus lactis,* which are two of the most common factitious hosts used in commercially rearing of *phytoseiid* predators. It has been shown that larger and slower species are more suitable as host species because they provide more food per unit of prey and are comparably easier to be caught by the predatory mite species. Thus, in a preferred embodiment of the invention the host species has a body length of 450 - 550 µm (male) and/or 500 - 700 µm (female).

In a further preferred embodiment of the mite composition the predatory mite species is selected from the group comprising the family *Phytoseiidae* (*Acarina*), preferably the species *Amblyseius andersoni, Neoseiulus californicus, Neoseiulus cucumeris* and *Amblyseius swirskii,* and most preferably *Typhlodromalus montdorensis.* These species have been shown to be especially suitable for cultivation together with the host species described above. One of the advantages of at least some of these species is that they have a comparably fast development time from egg to adult. Furthermore these species are especially suitable for controlling a wide variety of crop pests.

Use of the mite composition for controlling a crop pest is one preferred use of mite compositions as described herein.

In a further preferred embodiment of the mite composition, the composition further comprises a food source for the host species. Such a food source is only advantageous if the host species is a rearing population. Since the mite composition can also comprise a rearing population of a *phytoseiid* predatory mite species and at least one dead host species such a source for the host species is not needed in all embodiments of the present invention.

As described in more detail below it has been shown that at least for some host species a quite simple diet of wheat germ and/or wheat germ flakes, which are preferably contained in a carrier of sterilized bran is suitable. Such a simple diet has many advantages. First the preparation of such food is less time consuming and the workers are not burdened with the task of producing complex mixtures of food. From the prior art food mixtures are known which can include, dried fruit, buck wheat husks, flours, jam, special sugars and others. Since some of these nutriments are comparably costly, savings of costs is a second advantage of the simple diet. Thus, in a further preferred embodiment of the mite composition the food source comprises wheat germ and/or wheat germ flakes. In a further preferred embodiment these wheat germ and/or wheat germ flakes are in a carrier of sterilized bran.

The basic procedures involved in the commercial production of a phytoseiid predator mite, using either a diet of live or dead *Tyrolichus casei* mites.

An important aspect of the invention is a method for rearing a mite composition comprising a rearing population of a *phytoseiid* predatory mite species and at least one host species wherein the *phytoseiid* predatory mite species and at least one host species from the genus *Tyrolichus* are arranged together in a rearing unit. Examples and preferred embodiments of the method are described below.

Without exception, current commercial productions of any phytoseid species involve the same procedures, namely:
- selection of the astigmatid diet, and bulk production of this diet
- the selection and rearing of large quantities of the astigmatid mite, and
- the rearing and production of large quantities of the selected predator,
- using the selected astigmatid mite as live prey.

### The astigmatid diet and its bulk production

### 1. Selection

Since the astigmatid host *Tyrolichus casei* is recognized to be a 'stored food' mite, the diet will contain a mixture of some of the following ingredients - farinaceous foods such as flour, wheat germ, bran, together with dried yeast powder, sugars and other specific ingredients.
The selected materials will be mixed in a commercial food mixing machine.

### 2. Equilibration of the moisture content of the dry mix

Water is added to the mix in a proportion which will bring its moisture content (MC) into equilibrium with the relative humidity (RH) of the rearing rooms.

### 3. Physical environment of the rearing rooms

The rooms are fitted with sophisticated machines able to maintain the required conditions to plus or minus one °C and two to five % RH. In general, a physical environment of 75 to 85% relative humidity (RH), and a temperature between 21 to 25°C is most suitable for cultivation of the mite composition.
In a preferred embodiment of the method for rearing a mite composition the species from the order *Astigmata* is *Tyrolichus casei.*

### Rearing the astigmatid species - T. casei

### 1. Individual rearing containers and initial contents

Units are of a size for easy handling, and will require a 'breathable' sealed cover to allow an exchange of oxygen, preferably a gauze of 45 to 50 micron mesh.
Each will contain a quantity of the diet, plus a 'carrier' or bulking component. This usually consists of bran or granulated vermiculite sufficient to provide enough three dimensional space to allow the population freedom to expand, and is known as the substrate.

### 2. Addition of the mite population

The substrate of a matured unit is divided to give a starter colony for a new rearing unit.

The number of mites per litre of substrate is obtained using a method known as 'coning and quartering', described by Griffiths (1976).

It is then divided into parcels, depending on how many will be needed per litre to form the nucleus of the starter colony.
This number will depend on the intrinsic rate of increase (IRI) of the prey (that is the time taken to grow from an egg to an adult) and at what intervals mature units are required.

### 3. Rearing the units

A food supply is added to the starter colony, sufficient to feed the developing population until the density reaches the required level.

Moist vermiculite or bran will also be added to provide space into which the developing colony can expand.

New units are placed in the rearing rooms and allowed to develop to a high density, which usually takes no more than about ten days.

At maturity the cycle begins all over again, giving a re-stock starter colony or a supply of live prey to feed the predator starter colony.

In a further preferred embodiment of the method for rearing a mite composition the species from the order *Tyrolichus* is alive.

### Predator production using live T. casei as the prey

The steps in the rearing of predators are the same as defined above for the prey, except the prey is now the food for the predator.

### 1. Composition of the mature predator unit

The predator population of a mature unit will be at a high density. This will have been reached through feeding on the *T. casei* prey population, the level of which has been maintained at the right concentration, despite predator feeding, by the breeding of those *T. casei* fortunate enough not to have been eaten.
At this point the density of population will be determined as 1,000s (K's) per litre of substrate.

### 2. Producing a predator re-start unit or a sales unit

According to the determined density of the mature unit it will be divided into parcels of substrate, each containing sufficient predators needed to set up a re-start unit.

The size of this initial population is decided relevant to the estimated size it will reach at maturity, taking into account the IRI of the species.

### 3. Re-supplying a start-up unit

The initial predator population is placed in a clean start-up unit, to which is added a supply of food for maintaining the predator:
- a supply of live *Tyrolichus* population
- the parcel of predators, as determined in (2) above
- additional vermiculite or bran granules.

### 4. Obtaining the correct ratio of predator to prey during development through to maturity

The objective is to obtain an initial start-up ratio for good predator development which will remain stable throughout the period of the predator production.

This means calculating the amount of *Tyrolichus* population which needs to be added to achieve this ratio.

The predator or the prey must not be allowed to starve, nor must the prey be allowed to increase to a density which will interfere with predator development, especially egg laying. This task is the hardest to get right of all the various procedures.

Success relies heavily upon the knowledge and experience of a skilled person. Insufficient experience will necessitate the introduction of a mid- programme monitoring system to determine whether the ratio has skewed. This will add to the production costs. It is no exaggeration to say it can dictate the success or failure of a producer.

### Predator production using de-frosted, (dead) T. casei as the food

### 1. The supply of mites to be frozen

These come from exactly the same high density 'mature' units of live *Tyrolichus* mites as was used for re- stocking the predator start-up units.
A parcel of substrate (which contains the live prey population) is placed in suitably sized bags and put into deep freeze condition until completely frozen, where they can remain until required.

### 2. Automatic reduction in the farinaceous material

At the moment the *Tyrolichus* mature unit is selected for freezing the farinaceous yeast diet, originally present, will have been severely depleted by the feeding of the high density population of *Tyrolichus* throughout the development period.

What remains will be further diluted by the introduction of additional particles of inert vermiculite, at the time when the 'dead' diet is fed to a re-start predator unit. This reduction in farinaceous material reduces the risk of fungal infection.

### 3. Reduction in the RH of the rearing room

The *Phytoseiidae,* have a thick chitinised cuticle, and possess a breathing system which regulates the taking in of air from the atmosphere, unlike the *astigmatid* mites which breath through their very thin skin and are therefore sensitive to humidity changes. This enables the predators to live in a much drier atmosphere than the *Astigmata* which because they can only exchange oxygen through their cuticle (skin) need higher humidity conditions in order to survive and develop.

This means the predators can be reared at a RH atmosphere of 75 %, preferably never exceeding 80 %.

### 4. Addition of food to the re-start unit

Fresh amount of food, in the form of dead *Tyrolichus casei,* is introduced into a starter unit of predators, which will of course contain a known number of predators, (counted as described earlier) plus substrate and a depleted food supply, and then left to develop to the required density.

### Packing for sale and despatch to the crop owner

The mature units, for example of *Typhlodromalus montderensis,* will be sent to the packing department for processing. The concentration of predators per litre of substrate (vermiculite and/or bran particles) will be adjusted depending on the required rate of introduction into a particular crop for a particular pest species. Similarly, the containers into which the predators are placed and despatched vary according to destination and end usage.

### Physical and physiological characteristics

Preferred embodiments of the present invention are described in more detail below. These are only some of the possible examples which fall within the scope of the present invention and should not be interpreted as limiting the present invention.

In respect of these examples it has been found that this rarely encountered species - *Tyrolichus casei,* provides certain advantages which make it a preferred factitious host for cultivating *phytoseiid* predators. *Tyrolichus casei* provides certain advantages over other frequently used host species, particularly *Carpoglyphus lactis, Thyreophagus entomophagus* and *Tyrophagus putrescentiae.* These advantages are discussed below.

### Body size

The body size of the *Tyrolichus casei* species is much larger than that of the other three frequently used host species mentioned above, and compared with *C. lactis* and *T. putrescentiae* it moves much more sedately, so that in the very crowded environment of a rearing container it does little to disturb the equilibrium of the predator, allowing it to predate and breed with little hindrance.

It has been shown that predators provided with *T. casei* as prey will readily feed upon all preadult stages from egg to 2^{nd} nymph, as well as the adults in some cases. Thus, the *T. casei* population provides more nutrients per unit of prey in comparison to the other three *astigmatid* hosts mentioned above. Differences in body size between these species are compared in the following table showing data from Hughes (1976):

**Table 1: body size of different prey mite species.**

| Species | Body length [µm] | |
|---|---|---|
| | Male | Female |
| *T. casei* | 450 - 550 | 500 - 700 |
| *T. entomophagus* | 300 - 450 | 450 - 600 (long, thin) |
| *C. lactis* | 380 - 400 | 380 - 420 |
| *T. putrescentiae* | 280 - 350 | 320 - 415 |

### Prey in-balance (predator/prey ratios)

As mentioned above, the prey in-balance (predator/prey ratio) is crucial to the production of large numbers of predators. During the production cycle which is concerned with the rearing of the predator, two very different types of populations are involved simultaneously. Each of these populations is developing at the same time, in the same container, and both require to eat in order so to do. Since the factitious host is the food supply for the predator it is of major importance that, as the predator population grows, the number of prey individuals must also increase at a predetermined rate to sustain the desired predator/prey ratio.

Introducing a correctly defined initial ratio is crucial because, if one begins at start-up with a too high concentration of prey this will eventually give a density of bodies which will stifle the development of the predators. Conversely, if the concentration of prey is too low, it will cause them to starve. Thus, the ratio of predator to prey must be maintained throughout the predator production cycle, which is usually some seven, sometimes, ten days in duration.

Essential to achieving this objective is the need to set up the initial ratio correctly. Since the two rates of developments will invariably be different, the actual number of adults of both species present will need to be counted and the numbers adjusted according to the known intrinsic rate of development. Assessing the first and achieving the second is of major importance for each predator/prey-combination and especially for such a new combination as is the subject matter of the present invention.

Setting a wrong initial ratio can have serious consequences. Thus, at start-up the numbers of individuals (adults and nymphs, only) must be determined for each population by repetitive counts. These are made using the 'coning and quartering' technique of Griffiths (1976). The technique of assessing predator to prey ratios requires skill and concentration to carry out properly.

The use of *T. casei* has a distinct advantage over using other species such as for example, *T. entomophagus,* which is the only other favored factitious host currently being used in commercial productions of *T. montdorensis,* since due to the much larger size of *T. casei,* the increase in nutritive volume per body means, that less individuals are needed to satisfy predator feeding levels. This results in an easier, quicker counting and estimation of the correct initial ratio, as well as a significant reduction in materials.

Initial ratios which are preferred for cultivating *phytoseiid* predators are shown in the following table:

**Table 2: preferred initial ratios for cultivating phytoseiid predators.**

| Species | Preferred initial ratios |
|---|---|
| *T. casei* | 1:10 to 1:30 max |
| *T. entomophagus* | 1:50 to 1:100 max |

It follows from the above that during a commercial production using *T. casei,* a 5x reduction in volume will be achieved when compared to using *T. entomophagus.* This is most beneficial when handling and counting populations.

### A simple diet

Trials have shown a further unforeseen advantage of *T. casei* when used as prey for *phytoseiid* predators:
*T. casei* develops extremely well on a simple diet of wheat germ flakes contained in carrier of sterilized bran. Thus, in a further preferred embodiment of the method for rearing a mite composition the species from the genus *Tyrolichus* is fed with wheat germ and/or wheat germ flakes. This simple diet has the advantages that the workers are not burdened with the task of producing complex mixtures which can include, dried fruit, buck wheat husks, flours, jam, special sugars etc., which are often used to provide diets for factitious hosts. (e.g. as described in EP 07789077).

This simple diet eradicates problems of sticky lumps when sugars have to be added to the substrate. It allows the diet to be prepared quickly, even by unskilled persons. Thus, savings in both time and costs are possible.

Furthermore, the possibility of using a simple diet based on only wheat germ offers a solution to some problems for rearing predatory mites caused by the presence of different food mixtures:
- First, this food can be almost totally consumed and can stay attractive for the prey mite for a longer time. Using other mixtures containing yeast and/or sugar, dried fruit jam, etc., favours the development of bacteria and fungi.
- Secondly, very high populations of *T. casei* can be developed using minimal quantities of just wheat germ. Densities, in the range of 40 to 60 million individuals per liter, have routinely been achieved. This population density is much higher than the density of populations of *T. entomophagus* achieved in EP 07789077 productions.

These advantages, together with the need to produce significantly lower quantities of *T. casei* to feed the predators, results in a considerably reduced cost of production.

### Reductions in development times from egg to adult

A further considerable advantage of using *T. casei* as prey is the shortened development time from egg to adult under the regime of the physical parameters maintained in the constant temperature/humidity rearing rooms, on the simple diet mentioned above. The following table shows a comparison of development times for females of both *T. casei* and *T. montdorensis.*

**Table 3: Comparison of development times for females of T. casei and T. montdorensis.**

| Species | Time from egg to egg |
|---|---|
| *T. casei* | 7-8 days |
| *T. montdorensis* | 6-7 days |

Such short development times results in a shortened production cycle. In pilot production experiments, the cycle time has been reduced to seven days. In a preferred embodiment the host species has a development time from egg to adult and/or egg to egg of 8 days or less. In comparison with the data disclosed in EP 07789077 where the optimal production cycle for *T. entomophagus*/ *A. swirskii* is considered as extending up to 14 days. This provides significant savings.

In addition, big savings in time are vital to production of sales volumes during peak pest periods. Even in the absence of a peak pest period the method of the present invention allows for a quicker production time whilst at the same time producing very large numbers of *phytoseiid* predators.

### Production volumes

A further rewarding advantage of using *T. casei* as prey is related to the shortened development time from egg to adult. As shown in the following examples, also the production volumes of phytoseiid predators are increased significantly by using the species *Tyrolichus casei* as the factitious prey.

### Example 1

A series of cultures using the preferred combination of *T. montdorensis* and the factitious host *Tyrolichus casei* were setup following the rearing system as described above.

A production cycle of 7 days gave the following results (Table 4):

**Table 4: comparison of the population development in different rearing units after a production cycle of 7 days.**

| Unit | Set-up population (Nº of adults and nymphs/ litre) | Final population (Nº of adults and nymphs/ litre) | Increase factor |
|---|---|---|---|
| 1 | 128.000 | 435.200 | 3,40 |
| 2 | 128.000 | 396.800 | 3,10 |
| 3 | 128.000 | 422.400 | 3,30 |
| 4 | 153.600 | 384.000 | 2,50 |
| 5 | 153.600 | 358.400 | 2,33 |
| 6 | 153.600 | 409.600 | 2,67 |
| 7 | 128.000 | 435.200 | 3,40 |
| 8 | 128.000 | 396.800 | 3,10 |
| 9 | 128.000 | 345.600 | 2,70 |
| 10 | 140.800 | 409.600 | 2,91 |
| 11 | 140.800 | 409.600 | 2,91 |
| 12 | 140.800 | 422.400 | 3,00 |
| 13 | 115.200 | 396.800 | 3,44 |
| 14 | 115.200 | 371.200 | 3,22 |
| 15 | 115.200 | 448.000 | 3,89 |
| 16 | 128.000 | 422.400 | 3,30 |
| 17 | 128.000 | 396.800 | 3,10 |
| 18 | 128.000 | 371.200 | 2,90 |
| 19 | 115.200 | 448.000 | 3,89 |
| 20 | 115.200 | 409.600 | 3,56 |
| 21 | 115.200 | 435.200 | 3,78 |
| **Total mean** | **129.829** | **405.943** | **3.16** |

The total mean number of individuals of *T. montdorensis* developed after 7 days is much higher than the populations of other predatory species described in previous publications.

For example, in EP 2 048 941 a mean number of 93,088 individuals per litre of *Amblyseius swirskii* was achieved using the factitious prey *T. entomophagus,* and the mean number of the same predatory species was 70,625 individuals when using the factitious prey *C. lactis.*

### Example 2

A second pilot production trial to mass rear the predatory species *T. montdorensis* was setup using both the factitious prey *T. casei,* according to the methodology described in the present invention, and the factitious prey *C. lactis.* Compared to those obtained using *Carpoglyphus lactis* preliminary results using a production cycle of 7 days with *T. casei* as factitious host show significant advantages.

In a preferred embodiment of the method for rearing a mite composition, the mite composition is split every 6 - 8 days, preferably every 7 days.

As an example the populations of *T. montdorensis* were split every 7 days at different ratios according to the development of the populations. The populations were reared during four weeks (1, 2, 3 and 4). At the beginning of the trial, several mature units were mixed in a big container and then the material was divided into different starting units, so that all the starting units had the same initial densities of the predatory species, which was 448,000 individuals of *T. montdorensis*/ litre (before splitting). Half of these units were fed with *T. casei* and half with *C. lactis.* The populations that were fed with *T. casei* developed very well and therefore were split by three every week.

It has been found that in a preferred embodiment of the method for rearing a mite composition, the mite composition is split periodically by a factor of 2 - 4. In a further preferred embodiment of the method for rearing a mite composition, the mite composition is split periodically by a factor of 2,5 - 3,5, more preferably by a factor of about 3, even more preferably by a factor of 3.

In the example described above, this means that the total volume of the added food for the prey mite, the prey populations and the substrate was twice the initial volume before splitting. However, the populations that were fed with *C. lactis* were only split by 3 the first week and, because the populations did not develop properly, the ratio was reduced to 2.5, 2.5 and one in the following weeks number 2, 3 and 4, respectively.

The results obtained are presented in the following tables (Tables 5a - d):

**Table 5a: Comparison of the population development of T. montdorensis when reared together with the host species T. casei or C. lactis after week 1.**

| Week 1 | | | |
|---|---|---|---|
| Prey species | No. of predators (K's per litre) | | Increase factor |
| | initial set-up | at harvest | |
| *T. casei* | 149 | 435 | 2,92 |
| *C. lactis* | 149 | 332 | 2,23 |

**Table 5b: Comparison of the population development of T. montdorensis when reared together with the host species T. casei or C. lactis after week 2.**

| Week 2 | | | |
|---|---|---|---|
| Prey species | No. of predators (K's per litre) | | Increase factor |
| | initial set-up | at harvest | |
| *T. casei* | 145 | 474 | 3,27 |
| *C. lactis* | 133 | 256 | 1,92 |

**Table 5c: Comparison of the population development of T. montdorensis when reared together with the host species T. casei or C. lactis after week 3.**

| Week 3 | | | |
|---|---|---|---|
| Prey species | No. of predators (K's per litre) | | Increase factor |
| | initial set-up | at harvest | |
| *T. casei* | 158 | 489 | 3,09 |
| *C. lactis* | 102 | 77 | 0,75 |

**Table 5d: Comparison of the population development of T. montdorensis when reared together with the host species T. casei or C. lactis after week 4.**

| Week 4 | | | |
|---|---|---|---|
| Prey species | No. of predators (K's per litre) | | Increase factor |
| | initial set-up | at harvest | |
| *T. casei* | 163 | 538 | 3,30 |
| *C. lactis* | 77 | 51 | 0,66 |

### Example 3

Data from the pilot production trials according to the present invention have shown that using *T. casei* as the factitious host to rear *Amblyseius andersoni* also results in a big increase in production volumes. Compared to those obtained using *Carpoglyphus lactis* preliminary results using a production cycle of 7 days show significant advantages.

The methodology as described in the previous example (two) was followed using populations of the predatory species *Amblyseius andersoni* and the same two species as factitious prey species, *T. casei* and *C. lactis.* The populations of this predatory mite were reared during four consecutive weeks and they were split every 7 days. At the beginning of the trial, several mature units were mixed in a big container and then the material was divided into different starting units, so that all this starting units had the same initial densities of predatory species, which was 345,600 individuals of *A. andersoni*/ litre (before splitting).

Half of these units were fed with *T. casei* and half with *C. lactis.* The populations that were fed with *T. casei* developed very well and were split by 2.5 every week. However, the populations that were fed with *C. lactis* were only split by 2.5 the first week and, because the populations did not develop properly, the ratio was reduced to 1.5, 1.2 and to one in following weeks 2, 3 and 4, respectively.

The results obtained are presented in the following tables (Tables 6a - d):

**Table 6a: Comparison of the population development of A. andersoni when reared together with the host species T. casei or C. lactis after week 1.**

| Week 1 | | | |
|---|---|---|---|
| Prey species | No. of predators (K's per litre) | | Increase factor |
| | initial set-up | at harvest | |
| *T. casei* | 138 | 333 | 2,41 |
| *C. lactis* | 138 | 221 | 1,60 |

**Table 6b: Comparison of the population development of A. andersoni when reared together with the host species T. casei or C. lactis after week 2.**

| Week 2 | | | |
|---|---|---|---|
| Prey species | No. of predators (K's per litre) | | |
| | initial set-up | at harvest | Increase factor |
| *T. casei* | 133 | 346 | 2,60 |
| *C. lactis* | 147 | 206 | 1,40 |

**Table 6c: Comparison of the population development of A. andersoni when reared together with the host species T. casei or C. lactis after week 3.**

| Week 3 | | | |
|---|---|---|---|
| Prey species | No. of predators (K's per litre) | | |
| | initial set-up | at harvest | Increase factor |
| *T. casei* | 138 | 359 | 2,60 |
| *C. lactis* | 172 | 120 | 0,70 |

**Table 6d: Comparison of the population development of A. andersoni when reared together with the host species T. casei or C. lactis after week 4.**

| Week 4 | | | |
|---|---|---|---|
| Prey species | No. of predators K's per liter) | | |
| | initial set-up | at harvest | Increase factor |
| *T. casei* | 144 | 345 | 2,40 |
| *C. lactis* | 120 | 64 | 0,53 |

### Conclusions

It has been shown above, the selection of the rare and seldomly encountered species *Tyrolichus casei* as a new factitious host has provided a plurality of surprising advantages compared to the results obtained when using other factitious *astigmata.* The combination of all the advantages indicated above could not be anticipated by a person skilled in the art.

It is obvious that pairing a *phytoseiid predator* with suitable prey mites can still be improved.

In this respect it is considered that *T. casei* has the potential, not only to successfully partner the predator *T. montdorensis,* but also other predator species. Initial trials have shown this species has the potential to provide successful productions of *Amblyseius andersoni, A. swirskii, Neoseiulus cucumeris and N. californicus.*

Initial trials have shown this species also has the potential to provide successful productions of *A. andersoni,* and *Neoseiulus cucumeris.*

## Claims

1. Mite composition comprising a rearing population of a *phytoseiid* predatory mite species, one host species, wherein the host species is *Tyrolichus casei,* and optionally a food source for the host species,
**characterized in that**
the ratio for cultivating phytoseiid predators is 1:10 to 1:30 max or the host species is dead.

2. Mite composition according to claim 1,
**characterized in that**
the predatory mite species is selected from the family Phytoseiidae, preferably *Typhlodromalus montdorensis.*

3. Mite composition according to claim 1,
**characterized in that**
the predatory mite species is selected from the family Phytoseiidae, preferably selected from the group comprising the species *Amblyseius andersoni, Amblyseius swirskii, Neoseiulus cucumeris* and *Neoseiulus californicus.*

4. Mite composition according to any of the preceding claims,
**characterized in that**
the food source for the host species comprises wheat germ and/or wheat germ flakes.

5. Mite composition according to claim 4,
**characterized in that**
the wheat germ and/or wheat germ flakes are in a carrier of sterilized bran.

6. Mite composition according to any of the preceding claims,
**characterized in that**
the mite composition is arranged in a manageable sized rearing units, each sealed with a gauze of 45 to 50 µm mesh to allow an exchange of oxygen.

7. Use of the mite composition according to any of the preceding claims for controlling a crop pest.

8. Method for rearing a mite composition consisting of a rearing population of a *phytoseiid* predatory mite species, one host species and optionally a food source for the host species,
**characterized in that**
the *phytoseiid* predatory mite species and the host species *Tyrolichus casei* are arranged together in a rearing unit, wherein the ratio for cultivating phytoseiid predators is 1:10 to 1:30 max or the host species is dead.

9. Method for rearing a mite composition according to claim 8,
**characterized in that**
the species *Tyrolichus casei* is alive.

10. Method for rearing a mite composition according to claim 9,
**characterized in that**
the species *Tyrolichus casei* is fed with wheat germ and/or wheat germ flakes.

11. Method for rearing a mite composition according to any one of claims -8 or 9,
**characterized in that**
the mite composition is split every 5 - 15 days.

12. Method for rearing a mite composition according to claim 11,
**characterized in that**
the mite composition is split every 6 - 8 days.

13. Method for rearing a mite composition according to any one of claims 8 - 12,
**characterized in that**
the mite composition is split periodically by a factor between 1 - 5.

14. Method for rearing a mite composition according to claim 13,
**characterized in that**
the mite composition is split periodically by a factor between 2 - 4.

15. Method for rearing a mite composition according to any one of claims 8 - 14,
**characterized in that**
the mite composition is reared in a physical environment of 75 to 85% relative humidity (RH) and a temperature between 21 to 25°C.

## Patentansprüche

1. Milbenzusammensetzung umfassend eine Aufzuchtpopulation einer *Phytoseiid-*Raubmilbenart, eine Wirtsart, wobei die Wirtsart *Tyrolichus casei* ist, und optional eine Nahrungsquelle für die Wirtsart,
**dadurch gekennzeichnet, dass**
das Verhältnis zum Kultivieren von *Phytoseiid*-Räubern maximal 1:10 bis 1:30 ist oder die Wirtsart ist tot.

2. Milbenzusammensetzung nach Anspruch 1,
**dadurch gekennzeichnet, dass**
die Raubmilbenart ausgewählt ist aus der Familie *Phytoseiidae,* bevorzugt *Typhlodromalus montdorensis.*

3. Milbenzusammensetzung nach Anspruch 1,
**dadurch gekennzeichnet, dass**
die Raubmilbenart ausgewählt ist aus der Familie *Phytoseiidae,* bevorzugt ausgewählt aus der Gruppe umfassend die Arten *Amblyseius andersoni, Amblyseius swirskii, Neoseiulus cucumeris* und *Neoseiulus californicus.*

4. Milbenzusammensetzung nach einem der vorhergehenden Ansprüche,
**dadurch gekennzeichnet, dass**
die Nahrungsquelle für die Wirtsart Weizenkeim und/oder Weizenkeimflocken umfasst.

5. Milbenzusammensetzung nach Anspruch 4,
**dadurch gekennzeichnet, dass**
der Weizenkeim und/oder die Weizenkeimflocken in einem Träger sterilisierter Kleie ist/sind.

6. Milbenzusammensetzung nach einem der vorhergehenden Ansprüche,
**dadurch gekennzeichnet, dass**
die Milbenzusammensetzung in einer Aufzuchteinheit handlicher Größe angeordnet ist, jeweils verschlossen mit einem Mull mit 45 bis 50 µm Maschenweite, um einen Sauerstoffaustausch zu ermöglichen.

7. Verwendung der Milbenzusammensetzung nach einem der vorhergehenden Ansprüche zum Kontrollieren von Pflanzenschädlingen.

8. Verfahren zum Aufziehen einer Milbenzusammensetzung umfassend eine Aufzuchtpopulation einer *Phytoseiid*-Raubmilbenart, eine Wirtsart und optional eine Nahrungsquelle für die Wirtsart,
**dadurch gekennzeichnet, dass**
die *Phytoseiid*-Raubmilbenart und die Wirtsart *Tyrolichus casei* zusammen in einer Aufzuchteinheit angeordnet sind, wobei das Verhältnis zum Kultivieren von Phytoseiid-Räubern maximal 1:10 bis 1:30 ist oder die Wirtsart ist tot.

9. Verfahren zum Aufziehen einer Milbenzusammensetzung nach Anspruch 8,
**dadurch gekennzeichnet, dass**
die Art *Tyrolichus casei* am Leben ist.

10. Verfahren zum Aufziehen einer Milbenzusammensetzung nach Anspruch 9,
**dadurch gekennzeichnet, dass**
die Art *Tyrolichus casei* mit Weizenkeim und/oder Weizenkeimflocken gefüttert wird.

11. Verfahren zum Aufziehen einer Milbenzusammensetzung nach einem der Ansprüche 8 oder 9,
**dadurch gekennzeichnet, dass**
die Milbenzusammensetzung alle 5 - 15 Tage geteilt wird.

12. Verfahren zum Aufziehen einer Milbenzusammensetzung nach Anspruch 11,
**dadurch gekennzeichnet, dass**
die Milbenzusammensetzung alle 6 - 8 Tage geteilt wird.

13. Verfahren zum Aufziehen einer Milbenzusammensetzung nach einem der Ansprüche 8-12,
**dadurch gekennzeichnet, dass**
die Milbenzusammensetzung regelmäßig durch einen Faktor zwischen 1-5 geteilt wird.

14. Verfahren zum Aufziehen einer Milbenzusammensetzung nach Anspruch 13,
**dadurch gekennzeichnet, dass**
die Milbenzusammensetzung regelmäßig durch einen Faktor zwischen 2-4 geteilt wird.

15. Verfahren zum Aufziehen einer Milbenzusammensetzung nach einem der Ansprüche 8-14,
**dadurch gekennzeichnet, dass**
die Milbenzusammensetzung in einer physikalischen Umwelt mit 75 bis 85 % relativer Luftfeuchtigkeit (RH) und einer Temperatur zwischen 21 bis 25 °C aufgezogen wird.

## Revendications

1. Composition d'acariens comprenant une population d'élevage d'une espèce d'acarien prédateur *phytoseiid,* une espèce hôte, dans laquelle l'espèce hôte est *Tyrolichus casei,* et facultativement une source de nourriture pour l'espèce hôte,
**caractérisée par le fait que**
le rapport pour la culture de prédateurs *phytoseiid* est 1:10 à 1:30 maximum ou l'espèce hôte est morte.

2. Composition d'acariens selon la revendication 1,
**caractérisée par le fait que**
l'espèce d'acarien prédateur est choisie dans la famille des Phytoseiidae, de préférence *Typhlodromalus montdorensis.*

3. Composition d'acariens selon la revendication 1,
**caractérisée par le fait que**
l'espèce d'acarien prédateur est choisie dans la famille des Phytoseiidae, de préférence choisie dans le groupe comprenant l'espèce *Amblyseius andersoni, Amblyseius swirskii, Neoseiulus cucumeris* et *Neoseiulus californicus.*

4. Composition d'acariens selon l'une quelconque des revendications précédentes,
**caractérisée par le fait que**
la source de nourriture pour l'espèce hôte comprend des germes de blé et/ou des flocons de germes de blé.

5. Composition d'acariens selon la revendication 4,
**caractérisée par le fait que**
les germes de blé et/ou les flocons de germes de blé sont dans un support de son de blé stérilisé.

6. Composition d'acariens selon l'une quelconque des revendications précédentes,
**caractérisée par le fait que**
la composition d'acariens est disposée dans des unités d'élevage de taille gérable, chacune étant scellée par une gaze de 45 à 50 µm d'ouverture de maille pour permettre un échange d'oxygène.

7. Utilisation de la composition d'acariens selon l'une quelconque des revendications précédentes pour contrôler un parasite des cultures.

8. Procédé pour élever une composition d'acariens consistant en une population d'élevage d'une espèce d'acarien prédateur *phytoseiid,* une espèce hôte et facultativement une source de nourriture pour l'espèce hôte,
**caractérisé par le fait que**
l'espèce d'acarien prédateur *phytoseiid* et l'espèce hôte *Tyrolichus casei* sont disposées ensemble dans une unité d'élevage, le rapport pour la culture de prédateurs *phytoseiid* étant de 1:10 à 1:30 maximum ou l'espèce hôte étant morte.

9. Procédé pour élever une composition d'acariens selon la revendication 8,
**caractérisé par le fait que**
l'espèce *Tyrolichus casei* est vivante.

10. Procédé pour élever une composition d'acariens selon la revendication 9,
**caractérisé par le fait que**
l'espèce *Tyrolichus casei* est nourrie par des germes de blé et/ou des flocons de germes de blé.

11. Procédé pour élever une composition d'acariens selon l'une quelconque des revendications 8 ou 9,
**caractérisé par le fait que**
la composition d'acariens est divisée tous les 5-15 jours.

12. Procédé pour élever une composition d'acariens selon la revendication 11,
**caractérisé par le fait que**
la composition d'acariens est divisée tous les 6-8 jours.

13. Procédé pour élever une composition d'acariens selon l'une quelconque des revendications 8-12,
**caractérisé par le fait que**
la composition d'acariens est divisée périodiquement d'un facteur entre 1-5.

14. Procédé pour élever une composition d'acariens selon la revendication 13,
**caractérisé par le fait que**
la composition d'acariens est divisée périodiquement d'un facteur entre 2-4.

15. Procédé pour élever une composition d'acariens selon l'une quelconque des revendications 8-14,
**caractérisé par le fait que**
la composition d'acariens est élevée dans un environnement physique de 75 à 85 % d'humidité relative (HR) et d'une température entre 21 et 25°C.
